# EUROPEAN PATENT SPECIFICATION

(11) **EP 2 811 963 B1**
(45) Date of publication and mention of the grant of the patent: **08.07.2020**
(21) Application number: 13705031.6
(22) Date of filing: 06.02.2013
(51) Int. Cl.: A61H 7/00, A61B 5/103, A61F 5/14, A61H 1/00, A43B 7/14, A43B 3/00, A61H 23/00

(54) **DEVICE FOR REBALANCING THE PELVIS AND TRAINING METHOD ASSOCIATED THEREWITH**
VORRICHTUNG ZUR REKOMPENSATION DES BECKENS UND LERNVERFAHREN IM ZUSAMMENHANG DAMIT
DISPOSITIF DE RÉÉQUILIBRAGE DU BASSIN ET PROCÉDÉ D'ENTRAÎNEMENT ASSOCIÉ

(30) Priority: 07.02.2012 NL 1039360
(43) Date of publication of application: 17.12.2014
(73) Proprietor: Krullaards Technology B.V., 2264 AH Leidschendam (NL)
(72) Inventor: KRULLAARDS, Robert Leonard, 2264 AH Leidschendam (NL)
(74) Representative: Nederlandsch Octrooibureau
(86) International application number: PCT/NL2013/050065
(87) International publication number: WO 2013/119112

(56) References cited:
- WO-A1-00/74627
- JP-A- 2000 060 934
- JP-A- 2005 058 683
- JP-U- 63 077 041
- US-A- 1 981 379
- US-A- 2 619 729
- US-A1- 2006 282 018
- US-A1- 2008 083 416
- US-A1- 2010 094 184

## Description

### Field of the invention

The invention relates to a device for rebalancing the pelvis, comprising a support element, wherein the support element comprises a first side, the shape of which is adapted to rest on a base, and comprises a second side, the shape of which is such that when the first side rests on the base, a first elevation, a second elevation and an intermediate part are formed, wherein the first elevation is higher than the second elevation, the intermediate part provides a gradual transition from the first elevation to the second elevation, and the intermediate part comprises an upper surface which is inclined with respect to the base, wherein, in a position of use, the first side of the support element rests on the base and is positioned with respect to a foot of an individual standing on the base that the support element is situated underneath a large part of the arch of the foot in such a way that the first elevation is situated underneath a first foot arch part, which first foot arch part is situated in the centre on the inner side of the foot arch, and the second elevation is situated underneath a second foot arch part. The device may also be used for treating or preventing disorders resulting from an imbalance of the pelvis and the invention further relates to a training method associated therewith.

### Background of the invention

There are many disorders which are associated with an imbalance of the pelvis, such as disorders which are associated with organs in the vicinity of the pelvis, but also disorders which are associated with organs in the vicinity of the spinal column. The reason for this is that the balance or imbalance of the pelvis greatly affects the position of the spinal column. When the pelvis is not balanced, the spinal column is often also imbalanced. Balancing the pelvis may therefore result in balancing the spinal column, and thus prevent or treat disorders associated with organs in the vicinity of the spinal column. The inventor even has good reason to believe that disorders associated with the autonomous nervous system could also be the result of an imbalance of the pelvis.

Existing methods for treating or preventing disorders associated with the position of the spinal column relate, inter alia, to correcting the posture and immobilizing vertebrae with respect to each other by means of a surgical procedure. With these methods, the question whether or not the pelvis is balanced is barely considered, if at all. These methods make assumptions about the suitable position of the spinal column, which assumed position does not necessarily coincide with the intrinsically correct position of the spinal column, that is to say the position which is most advantageous for the specific individual and which is accompanied by a balanced pelvis.

Known methods and devices for treating and preventing disorders which, inter alia, relate to the position of the spinal column are aimed at stimulating pressure points in the feet. A known method relates to acupressure of the feet, in order words to influencing energy pathways, for example energy pathways running through the feet. With such methods, it may be possible to alleviate symptoms of the above-described disorders for a short period of time, but the effect does not last as the method does not lead to a changed position of the pelvis and/or the spinal column.

A device which relates to the latter method is known from CN 2181930 (Y). The aim of this device is to massage or stimulate the foot arch by means of structures which are present on an elevation. This elevation is such that, in most cases, it fits underneath the arch of the foot without significantly raising the foot arch.

WO 0074627 (A1), which forms the closest prior art and the basis for the preamble of claim 1, discloses a device in which an elevation exerts some pressure on a central part in the inner side of a foot arch and may in this case raise it, depending on the shape of the foot. This document cites a maximum height for the support element of at most 1.9 cm. In this case, the pressure exerted on the foot arch is relatively low and is distributed more or less evenly across the entire foot arch. Furthermore, this patent document states that the object of the device is to improve the architecture of the foot in order to thus improve the state of the rest of the body. Such a device is therefore intended in particular for individuals who have feet of which the shape is not optimal, for example flat-footed individuals. For an average normal foot, the height of this elevation is substantially equal to the space between the ground and the foot arch. This device is thus intended specifically for stimulating a good position of the foot.

Other devices comprising elevations for massaging the foot arch are known from JP2005058683 (A) and KR100835206 (B1).

US1981379 discloses a device with spherical elevations. The aim of such a device is to rearrange the bones in the foot by means of the high elevations, so that they return to their natural position. The spherical elevation pushes the bones apart. Therefore, standing on such a device cannot be very comfortable and will even be painful. An individual standing on such a device can therefore not relax to such a degree that the muscles in the body can pass from a tense state to a relaxed state in order to improve the position of the pelvis (see below for more details about the action of the device according to the present invention). It is even the aim of said device described in US1981379 to exercise muscles, in particular the muscles in the feet and legs, in other words to tighten them, so that these muscles are strengthened. No mention is made of effects on disorders which may be associated with an imbalance of the pelvis.

Other devices with an elevation for the foot arch include arch supports. The elevations in such devices are aimed at supporting the foot arch in a position which is desirable and slightly natural for the foot. The elevation has a shape resembling that of a space between a base and a desirable position of a foot arch. This desirable position is a natural position and does not have any additional, therapeutic aim. That part of the surface of an arch support which makes contact with a foot arch does not have a surface which is inclined to such an extent that a certain part of the foot arch is clearly raised more than another part of the foot arch situated nearby. The maximum incline of the above-described surface of the arch support is approximately 25 degrees. The arch support will exert a substantially even pressure on the entire foot arch. An arch support will only result in an elevation of the foot arch in the case of flatfooted individuals and this elevation will be distributed evenly across the foot arch.

No device is known which comprises elevations intended for the foot arch in which a specific area of the foot arch is raised significantly in order to produce a therapeutic effect and in which another area of the foot arch is only partly supported in order to produce a stable and pleasant position and thus to relax certain muscles and/or groups of muscles. Furthermore, no device is known which comprises elevations intended for the foot arch and which has been shown to have an advantageous effect on the position of the pelvis and on disorders resulting from an imbalance of the pelvis. A device which can treat or prevent said disorders in a relatively simple manner is therefore also desirable.

### Summary of the invention

To this end, the invention provides a device for rebalancing the pelvis according to claim 1.

In this application, the term "pelvis" is understood to mean the entirety of the two pelvis halves and the tailbone in between. Rebalancing of the pelvis is intended to include training and retraining of the muscles in the pelvic and lumbar regions and also realignment and repositioning of the bones and muscles in the pelvic and lumbar regions. It is also intended to include treating or preventing disorders resulting from an imbalance of the pelvis.

The inventor has empirically found that standing on a base in which a device as described above is in a position of use has a positive effect on the balance of the pelvis and consequently has a positive effect on the abovementioned disorders. There are many known disorders which are associated with an imbalance of the pelvis, such as disorders associated with organs in the vicinity of the pelvis, but likewise disorders which are associated with organs in the vicinity of the spinal column. As has already been mentioned above, the balance or imbalance of the pelvis has a significant effect on the position of the spinal column. Balancing the pelvis may therefore lead to the spinal column becoming balanced, and thus prevent or treat disorders associated with organs in the vicinity of the spinal column. Some examples of the above-described disorders are gallbladder disorders, liver disorders, stomach disorders, kidney problems, diabetes, cardiac arrhythmia, impaired lung function, rheumatism, some forms of sterility, constipation, colitis, varicose veins, incontinence, impotence, low back pain, poor circulation in the legs, disorders in the sacroiliac region, trapped nerves and associated pain and the like. In addition, the inventor has found that the device according to the invention can successfully treat disorders associated with the autonomous nervous system, such as impaired secretion of sweat, impaired circulation, skin disorders, such as eczema and psoriasis and the like and can also assist in the normalisation of blood pressure.

This positive effect on the abovementioned disorders is seen in particular after an individual has stood on the support element in a suitable manner and has, optionally, bent both legs slightly, with the heel staying on the ground during bending and the arch remaining in contact with the support element.

If the individual assumes a position which differs from the upright position, for example a recumbent position, the support element may have the same effect if it is held against the foot arch.

The theory on which this is based is that by significantly raising the plantar fascia or a part thereof and supporting another part of the foot in such a manner that an individual can stand on the support element in a relaxed position, a certain muscle, in particular the ilio psoas muscle or muscle group relaxes, which muscle or muscle group had previously made a balanced pelvis impossible. By relaxing these one or more muscles, the pelvis, and subsequently possibly the spinal column, is balanced within a short period of time, usually within 15 seconds.

When the spinal column and/or the pelvis are in an intrinsically good position, that is to say are in balance, nerve bundles running along the spinal column and/or the pelvis function better. This has a positive effect on organs or tissues which are connected to the spinal column by means of these nerve bundles. The position of the pelvis can be measured before and after an individual has stood on the device.

The function of the first elevation is specifically to raise the first foot arch part by said elevation pressing into the first foot arch part. This pressure could result in an unpleasant pressure sensation and could possibly bring the individual out of balance or could result in cramp or excessive tension in a muscle. The function of the second elevation and/or the intermediate part is to prevent this from happening. The latter parts enable the individual to assume a stable and relaxed position while the first foot arch part is being raised. This stable and relaxed position is important for the operation of the device, as has been described above.

The term "foot" refers to a foot of an adult or child. The term "individual" refers to a human being.

The range of the elevation of the first foot arch part should be about 0.8 - 3 cm with respect to the position when standing on a flat base. This value refers to an adult foot and also to a foot of a child. Another way of indicating the elevation in which the length of the foot is given more consideration is 0.07 to 0.12 times the length of the foot. The support element which is adapted to an adult foot with a length of 25 cm may, for example, result in an elevation of the first foot arch part in the range of 1.75 to 3 cm and a support element which is adapted to the foot of a child of 16 cm may, for example, result in an elevation of the first foot arch part in the range from 1.12 to 1.92. The second elevation may increase the height by 0.05 to 0.4 cm compared to the position of the second foot arch part if the individual were to stand a flat surface.

The term "foot arch" is understood to mean that part of a foot which is adjacent to the space between the foot and the ground, which space is formed when the foot stands on said ground.

In the position of use, the first elevation is preferably substantially rigid, so that it can withstand the relatively large forces of the individual who stands on the support element and can raise the first foot arch part. The upper surface of the first elevation which is turned towards the foot arch in the position of use is preferably a sloping surface, if desired said surface may have a soft cover. Such a soft cover may assist in retaining the foot on the first elevation and prevent it from sliding downwards. The upper surface of the first elevation may also have a friction increasing surface such as a rubberised surface or a ridged surface on which a bare or socked foot can grip.

The support element is preferably made for a substantially normal foot. For more flatfooted feet or high-arched feet, the first elevation will have to have different dimensions. For these kinds of feet, the first elevation has to be made higher or lower, respectively. This indicated the difference between a support element according to the present invention and an arch support. With an arch support, the desired position of the foot is preferably the position of a healthy or average foot. The height of the elevation for the foot arch is thus more or less the same in the case of an arch support, and more or less independent of the shape of the foot, while the height of the first elevation of the support element according to the present invention, by contrast, has to be adapted to the shape of the foot in order to achieve a similar effect with differently shaped feet. Another difference is that the upper surface of an arch support follows the shape of the foot arch, and the shape of the upper surface of the second side of the support element according to the present invention, by contrast, deviates from that of a foot arch. It will be understood that although reference is given to a first elevation, second elevation and an intermediate part, these parts of the surface may be continuous and each support element may be a single continuous surface.

A preferred embodiment of the device according to the present invention comprises deforming means for moulding the support element from a deformable part, such as using pneumatic or hydraulic means.

The deformable part may have all the required properties of a support element and may furthermore be shaped in order to make the support element fit a foot, but it may also be a part which does not yet have the required properties of a support element, but is shaped to form a support element by the deforming means. Preferably, the deformable part is made from flexible material, so that the shaping results in a support element having a slightly sloping upper surface. If desired, the first elevation, the intermediate part and/or the second elevation may be shaped separately from each other. The device may be designed in such a way that the deforming means can produce the optimum support element for the respective foot. If desired, the shaping means are coupled to a signal from the pressure sensors or from the weighing means, which signal indicates that the tilted position of the pelvis has been remedied or which signal indicates a certain threshold value. The signal may be audible, visual or tactile. The shaping means are preferably capable of shaping the support elements independently from each other.

In another preferred embodiment of the device according to the present invention, a spacer is provided between the two support elements. The spacer may be between 0.5-15 cm in width and the support elements are positioned in such a manner that the first elevations are directed towards one another and the first elevations, the intermediate parts and the second elevations are substantially in line. By experimenting, the inventor has found that the desired result is not achieved if these parts of the support elements are not substantially in line and are, for example, positioned in such a way that the individual has to stand with one foot in front of the other foot or with his/her feet in a V shape. In other words, the individual should be able to stand on the support elements with the medial edges of the feet substantially parallel to each other.

With regard to treatment, it is important that the individual is able to stand in a stable position. The spacer should preferably provide the correct distance. A distance of 4 cm, for example, is suitable for a child, while a distance of 8 cm is suitable for an adult man. By experimenting, the inventor has determined that if this distance is greater, the desired effect is not achieved.

There may be provided engagement means to retain the support elements to the base and to each other or to the spacer. It will be understood that the support elements may rest directly on the base or may be carried indirectly by an intermediate member or support plate. The engagement means may comprise recesses which cooperate with each other, but, if desired, also hook and loop fasteners or other connecting means. Alternatively, the support elements may be integrally formed as a single piece with each other or with the spacer and/or with the base.

The engagement of the support elements with the spacer may also be such that it prevents the support elements from being pushed towards each other by the weight of the individual and from no longer being able to exert their function properly.

The spacer preferably comprises two supporting walls which, in the position of use, run transversely to the base for supporting the medial sides of both feet which face one another. As a result of these supporting walls, the individual can determine the correct position on the support elements more accurately.

The support elements may also be detachable and may subsequently be used as the inner sole of a shoe. Thus, the support element according to the present invention can be fitted in an optionally adapted shoe.

The plate is particularly useful to achieve a standardized arrangement of the support elements. The support elements may be fixedly attached to the plate. Alternatively, the bodies can be releasably attached to the plate, so that the type of support element can be adapted to the foot of the individual. Support elements which are adapted to children, to flatfooted adults and the like may be attached to the plate as desired. Preferably, the distance between the bodies can be adjusted in accordance with the type of support element. Preferably, two support elements, a spacer and engagement means are connected to the plate, so that, upon engagement, the distance between the two support elements is 0.5-15 cm and the support elements are positioned in such a manner that the first elevations face each other.

The plate and the support elements may form one single piece. The first side of the support element is then an imaginary plane through the device, which imaginary plane runs parallel to the top surface of the plate and with respect to which the support element forms the elevations and the intermediate part. The top surface of the plate and of the support elements may be made in one piece.

In yet another preferred embodiment of the device according to the present invention, a pressure sensor is incorporated at least in the first elevation.

This has the advantage that it is possible to monitor whether the individual is standing on the support element correctly. The pressure sensor may be merely a pressure switch giving an on-off reading or may even be a proximity sensor. Preferably, the pressure sensor can determine a value of the pressure. Determining a pressure value can give an indication of the shape of the foot and of the state of the pelvis - balanced or not balanced - or the spinal column. Obviously, in order to achieve this, certain data about the individual has to be known, such as the weight and the like.

The support element comprises several pressure sensors, all of which can preferably determine a pressure value in order to offer an impression of the pressure distribution in the foot arch while the individual stands on the device, optionally in a suitable manner. To the knowledge of the inventor, no device is yet known in which the pressure is measured at that part of the foot which normally does not touch the floor. The device preferably comprises a memory to be able to store these pressure measurements. A treatment may comprise several sessions, in which a pressure value may be determined each time. Determining the pressure value may be useful in order to follow the course of the treatment. The electronic parts for the one or more pressure sensors and an optional memory, as described above, are preferably accommodated in the spacer. It is preferable that the device also comprises a electronic control unit for determining the difference between the measured values of the different pressure sensors on the left and right. The fact is that an imbalance in the pelvis is usually accompanied by a tilted position. An equal pressure distribution on the left and right could mean that the pelvis is in balance. If desired, a signal could be emitted if the latter is the case. The signal may be audible, visual or tactile. The individual and the practitioner then know that the device has had a positive effect on the balance of the pelvis.

The device also comprises weighing means. The advantage thereof is that the function of scales, i.e. determining the body weight, can be combined with treating or preventing said disorders. In addition, data about the weight of an individual is important in relation to the height of the foot arch and a possible determination of the pressure value. The aforementioned values can be used to diagnose a disorder or condition and to follow an individual during a treatment. The weighing means can give a relative value, e.g. between individual feet or between parts of the feet. Alternatively and preferably, it can give absolute weight values, whereby the sum of the individual weights equals to the actual weight of the patient.

The device comprises a first support element, a second support element, and a first plate part and a second plate part for forming the base for the first support element and a first foot, and the second support element and a second foot, respectively, wherein the device also comprises weighing means, wherein the weighing means comprise a first weighing unit and a second weighing unit which are operatively connected to the first plate part and the second plate part, respectively. This means that the first weighing unit weighs that part of the body which rests on the one foot and the second weighing unit weighs that part of the body which rests on the other foot in such a way that the weights, taken together, form the weight of the individual. If these weights are not equal, then this indicates a tilted position of the pelvis. The advantage of the preferred embodiment comprising the two weighing units is that this tilted position can be expressed in different values in each weighing unit, that is to say that the one weighing unit indicates a greater weight than the other weighing unit. When the values of both weighing units become equal, this is an indication that the pelvis is in balance and that the tilted position has been remedied. If desired, a signal can be emitted to indicate when this is the case. The individual and the practitioner then know that the device has had a positive effect on the balance of the pelvis. Preferably, the spacer is provided with a display for indicating the different values.

Preferably, the device also comprises a height sensor for determining the position of the first foot arch part with respect to a flat surface. A height sensor may, together with one or more pressure sensors, be important for diagnosing the abovementioned disorders. In addition, by determining the above-described height, it is possible to consider which support element is most suitable.

According to a most important aspect of the invention, the device is also provided with feedback to a user regarding the correct location of the feet on the support elements. As indicated above, this feedback may be responsive to pressure or contact sensors indicating both the location and pressure of the feet and/or arches on the support elements. This feedback may be audible, tactile or visual. Most preferably, the feedback is audible and comprises a signal in the region of 50 - 5000 Hz, more preferably between 660 Hz and 3850 Hz and most preferably in the 2800 - 2900 Hz region. Without wishing to be bound by theory, it is believed that the perception of signals in this region is fed back via the pre-motoric cortex to the cortico-spinal path, causing relaxation of the muscles surrounding the spinal column. This has a particular effect on relaxing the musculus psoas whereby the pelvis assumes its natural position and nerves between the vertebrae are freed. The use of audible feedback is also believed to provide a stabilising effect on blood pressure. The audible signal may be generated for as long as the user maintains the feet in the correct position on the support elements. Alternatively, it may continue for a given period and terminate once the treatment is complete, indicating to the user that they may step away.

The device also preferably comprises vibrating means for causing the support element to vibrate. Vibration of the support element can have a positive effect on the action thereof, by providing exteroceptive feedback through the feet of the user, in particular, that part of the foot that is in contact with the support elements. The vibration may be initiated by correct positioning of the feet as described above. It may continue for a given period indicating the required duration of the treatment. Preferably the vibration is generated by an eccentric mounted motor or the like

Just as determining the above-described values is important, so is the determination of the body temperature. The device according to the present invention therefore preferably also comprises means for determining the body temperature, preferably the body temperature in the foot, in order to be able to give an indication of the blood flow in the foot. The means for determining the body temperature may be based on infra-red radiation, in which case no contact is made with the body, or may be based on contact sensors or on a material whose colour changes if the temperature changes.

According to the invention, the device comprises an electronic control unit for controlling operation of the device including a memory for storing device parameters and treatment parameters. The control unit may be located in the base or in the spacer or may be provided remotely. The device may also include a communication port for communicating with a remote computer, which may serve as the control unit or which may be used for additional functions such as data storage and analysis.

According to a preferred configuration, the electronic control unit may be programmed to follow a treatment protocol comprising: displaying respective weight readings when a user initially stands on the base with feet spaced apart; emitting an audible signal when a user stands on the base with arches in contact with the support elements; providing a tactile vibration to the feet during a period of at least 5 seconds while a user alternately bends a first and second knee four times, while keeping the arch in contact with the support elements. By monitoring and guiding the use of the device in this manner, a user is provided with an indication of correct use of the device. Bending the left and right knees alternately and twice each in this manner causes the desired release of the pelvis and relaxation discussed above. The contact with the arch is monitored by the use of pressure sensors or other suitable contact or proximity sensors as discussed above.

The invention also concerns a device for rebalancing the pelvis, comprising a base and first and second support elements mounted on the base, the support elements, comprising contoured surfaces arranged to support beneath the arches of a user's feet when standing on the support elements with the medial edges of the feet generally parallel and spaced by between 4 cm and 15 cm, the device further comprising sensors for determining a contact between the arch and the contoured surface and an indicator operatively connected to the sensors to provide feedback of contact between the arch and the contoured surface, indicative of a correct position of the feet . This device may be provided with all or any of the features as described above.

The device may comprise just a simple base plate carrying the support elements or may also form part of a larger structure. The larger structure may comprise a hand rail at a height above the base for gripping by a user while standing on the base, The hand rail is preferably attached to the base and may serve as a location for supporting a display panel. The base may be of a size to receive just two support elements or may be larger, comprising other zones for performing other measurement and/or treatment functions. In one particular embodiment, the base carries two plates, a measurement plate and a treatment plate, at least the later being provided with the support elements.

The invention also relates to a method of personal training using a device as described above, the method comprising: standing on the base with the heels spaced apart and out of contact with the support elements; measuring a differential heel pressure for the respective heels and displaying a representation of the difference; standing on the support elements with the feet close together and the medial edges generally parallel to each other such that the arches enter into contact with the first elevation, the second elevation and the intermediate part of the support elements; alternately bending a first and second knee at least four times while keeping the arch in contact with the support elements; and stepping off the support elements.

The method may further comprise emitting an audible signal when the user's arches are in correct contact with the support elements. Additionally or alternatively, the method may further comprise providing a tactile vibration during a period of at least 5 seconds while the user performs the knee bending motions and the arches remain in contact with the support elements.

### Brief description of the figures

The invention will be described below with reference to a non-limiting exemplary embodiment of the device according to the invention, in which:
- Fig. 1: shows a diagrammatic representation of a plan view of a support element;
- Fig. 2: shows a diagrammatic representation of a cross section through the support element along the line A-A from Fig. 1;
- Fig. 3: shows a diagrammatic representation of a cross section through the support element along the line B-B from Fig. 1;
- Fig. 4: shows a diagrammatic representation of a plan view of a support element in which the positions of pressure sensors are shown;
- Fig. 5: shows a diagrammatic representation of a cross section through the device comprising a plate and a spacer, similar to the cross section in Fig. 3;
- Fig. 6: shows a diagrammatic representation of an underside of a foot;
- Figs. 7 A-F: show a diagrammatic representation of a view of a foot standing or not standing on a prior-art device and a device according to the present invention, respectively, and the respective devices themselves;
- Figs. 8 A-D: show a diagrammatic representation of a cross section of a foot, standing on a prior-art device and a device according to the present invention, respectively, and standing on a base without a device;
- Fig. 9: shows a bar graph which plots the mean tilted position of the pelvis and the difference in effect of an arch support (C) and a support element (D) with regard to this tilted position;
- Fig. 10: shows a second embodiment of the invention in perspective view;
- Fig. 11: shows the second embodiment of Fig. 10 in exploded view;
- Fig. 12: shows an installation according to a third embodiment of the invention; and
- Fig. 13: shows a bar graph of average blood flow rate in the posterior tibial artery for a test group of individual users of the device of Fig. 10.

### Description of preferred embodiments of the invention

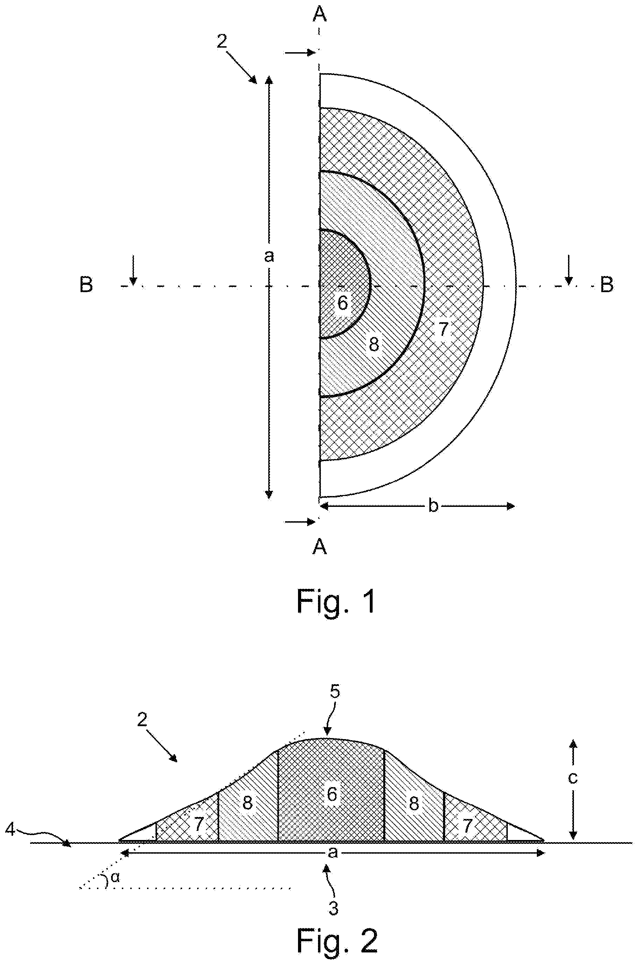

Fig. 1 shows a plan view of the second side (5) of a support element (2) of a preferred embodiment of a device according to the present invention. In this figure, the arrangement of the first elevation (6), the second elevation (7) and the intermediate part (8) and the mutual relationship of their upper surfaces are shown. The plan view of the support element (2) is in the shape of a semicircle, with the first elevation (6) being situated in the centre, and the intermediate part (8) extends radially from the first elevation (6) and the second elevation (7) extends radially from the intermediate part (8). In this embodiment, the dimension denoted by "a" is approximately 10 cm in real life. In this embodiment, the dimension denoted by "b" is approximately 6 cm in real life.

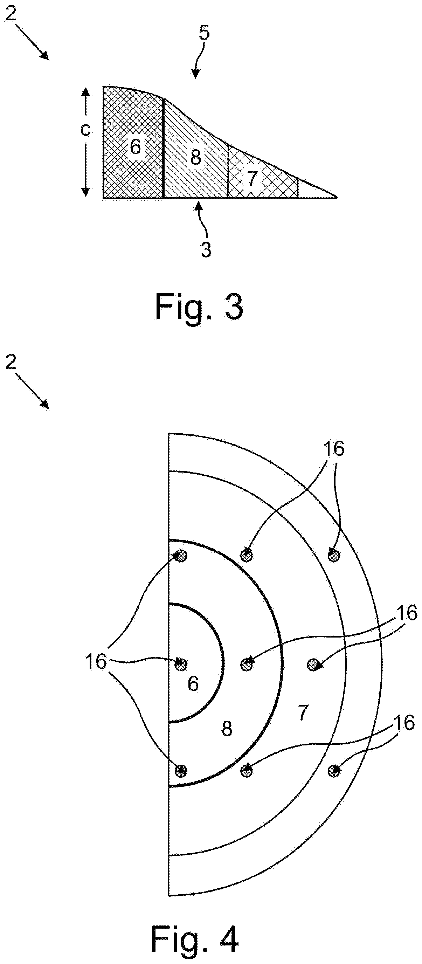

Figs. 2 and 3 show the relative heights of the first elevation (6), the second elevation (7) and the intermediate part (8) relative to a first side (3). In Fig. 2, the angle between the tangent line of the upper surface of the intermediate part (8) and the base is indicated by means of a dotted line. It can be seen that the angle of inclination (α) of the upper surface of the intermediate part (8) is approximately 35 degrees. It can also be seen in Fig. 2 that,

in this embodiment, the angle of inclination of the upper surface of the edge of the support element (2) and a part of the second elevation (7) with respect to the base is slightly more acute than the angle of inclination mentioned above and denoted by α. In this embodiment, the dimension denoted by "c" is approximately 3 cm in real life.

Fig. 4 shows a plan view as in Fig. 1, in which the position of pressure sensors (16) is shown. Pressure sensors (16) positioned in this way can give information about the position of the individual on the support element (2) and about the shape of the foot which is on the support element (2).

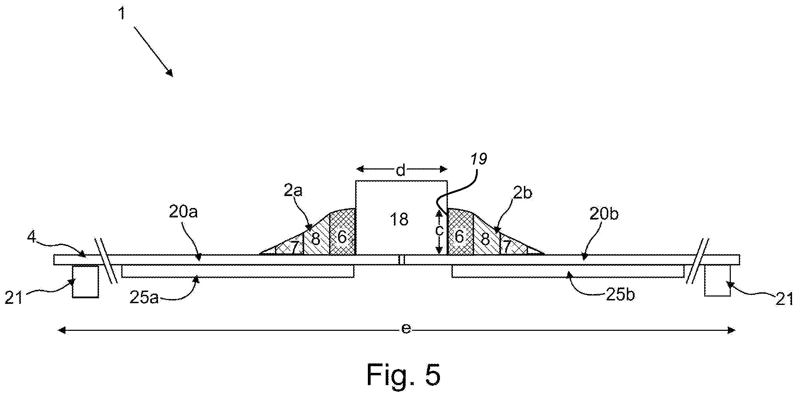

Fig. 5 shows a preferred embodiment of a device (1) comprising a first support element (2a), a second support element (2b), and a first plate part (20a) and a second plate part (20b) for forming the base for the first support element (2a) and a first foot, and the second support element (2b) and a second foot, respectively. Between the two support elements (2a and 2b) which are positioned in such a way that the first elevations (6) face each other, a spacer (18) is present. This preferred embodiment is developed in such a way that engagement means (19) connect the sides of the support elements (2) which run substantially at right angles to the base (4) in a simple manner to the sides of the spacer (18), for example using adhesive means. The spacer (18) creates a distance, denoted by "d", which is approximately 5 cm in real life. In this embodiment, the dimension denoted by "e" between an edge of the plate (20) and the other opposite edge of the plate (20), across the support elements (2), is approximately 50 cm in real life; sufficiently large for two feet which are a distance of approximately 5 cm apart. It can be seen that the plate (20) rests on small feet (21). This embodiment also comprises weighing means, wherein the weighing means comprise a first weighing unit (25a) and a second weighing unit (25b) which are operatively connected to the first plate part (20a) and the second plate part (20b), respectively.

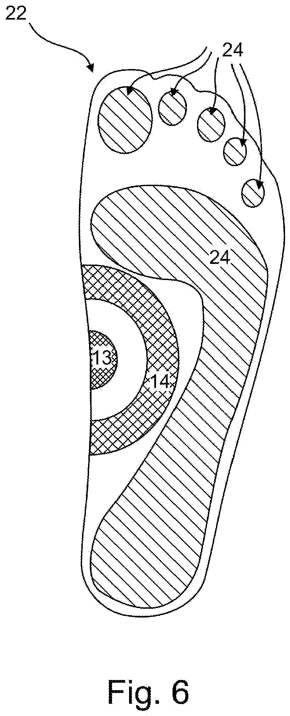

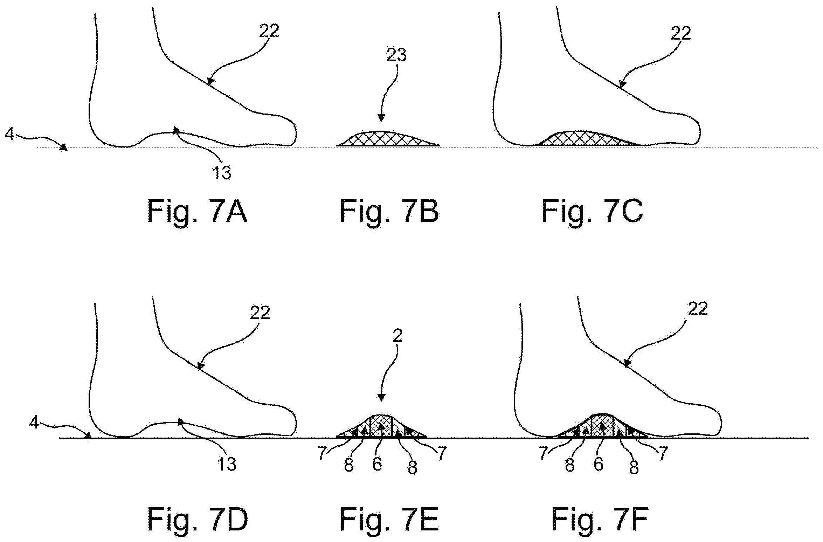

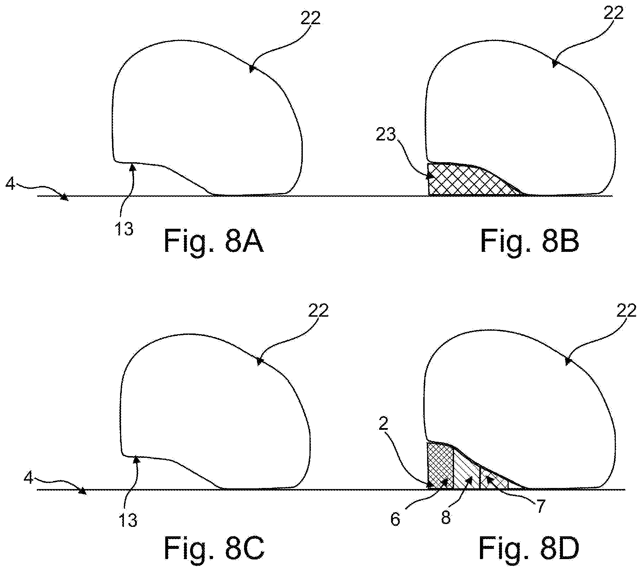

Fig. 6 shows an underside of a foot (22) and indicates where the first foot arch part (13) is approximately situated, which first foot arch part (13) is raised by the first elevation 0.8 - 3 cm with respect to the position of the first foot arch part (13) if the individual stands on the base without the support element being in its position of use, and where the second foot arch part (14) is approximately situated, which second foot arch part (14) is raised by 0.05 to 0.4 cm with respect to the position of the second foot arch part (14) if the individual stands on the base without the support element (2) being in its position of use, and that part of the underside of the foot which normally always rests on the ground when the foot is standing on the ground (24). The abovementioned elevations are such that the first foot arch part (13) is raised substantially, as a result of which this part is subjected to a relatively significant pressure, and the second foot arch part (14) is supported and does not experience any significant high pressure compared to the remaining part of the foot which rests on the ground. The area between the first foot arch part (13) and the second foot arch part (14) is an area where the elevation gradually runs from the elevation of the first elevation to the elevation of the second elevation, as can also be seen in Figs. 7 and 8.

Figs. 7 A-F show the difference between a prior-art device (23), for example an arch support, and a preferred embodiment of the device according to the present invention, regarding the elevation of the first foot arch part (13), in other words a central part on the inner side of the foot arch of a foot (22). Fig. 7B and 7E show that the maximum height of the support element (2) according to the present invention is relatively higher than the prior-art device (23). The intention is to show a foot (22) in a normal, healthy position. As can be seen in Figs. 7A-C, the prior-art device (23) supports the foot arch and keeps this in its normal, healthy position. Fig. 7F, in which the foot (22) of an individual stands on the support element (2) according to the present invention, clearly shows that the first foot arch part (13), in other words the central part on the inner side of the foot arch (13), is raised. In this position of the foot (22), the plantar fascia will be pushed in. It can also be seen that the foot arch is supported again in the direction of the front and rear side of the foot (22), similar to the prior-art device, as can be seen in Fig. 7C. It can be seen in Fig. 7F that the first elevation (6) and the intermediate part (8) raise the foot arch and the second elevation (7) supports the foot arch.

Figs. 8A, 8B, 8C and 8D show cross sections of a foot (22) at right angles to the direction in which the foot (22) extends and at the location of the first foot arch part, that is to say approximately the centre of the foot, see also Fig. 6. Figs. 8A and 8C show the shape of the foot (22) of an individual when the latter is standing on the ground without any other device being situated underneath the foot arch. The intention is to show a foot (22) in a normal, healthy position. The prior-art device (23), such as for example an arch support, supports the foot arch and keeps it in its normal, healthy position. Fig. 8D, in which the foot (22) of an individual is situated on the support element (2) according to the present invention, clearly shows that the first foot arch part (13) is raised. It can also be seen that the foot arch is supported again in the direction of the lateral side of the foot (22), similar to the prior-art device, shown in Fig. 8B. It can be seen in Fig. 8D that the first elevation (6) clearly raises the foot arch and the second elevation (7) supports the foot arch.

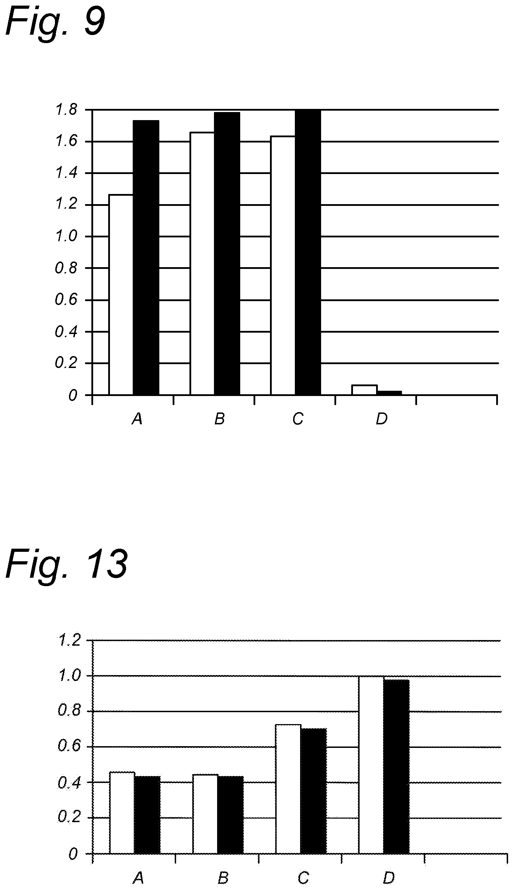

Fig. 9 shows a bar graph which plots the mean tilted position, in which the tilted position is measured using a Palm pelvis meter, as described in "The Accuracy of the Palpation Meter (PALM) for Measuring Pelvic Crest Height Difference and Leg Length Discrepancy", Journal of Orthopaedic & Sports Physical Therapy, vol 23, no. 6, June 2003. The left-hand bars represent the mean values of the tilted position for a group of 14 men and the right-hand bars represent the mean values of the tilted position for a group of 16 women. 'A' denotes the mean tilted position of the group of women and men when they stand on the ground with shoes. 'B' denotes the mean tilted position of the group of women and men when they stand on the ground without shoes. The group of men and women have stood on arch supports for 15 seconds. The effect thereof on the tilted position is represented by 'C'. It can be seen that this has had no effect. The bars denoted by 'D' illustrate the mean tilted position after the group has stood on a device according to the invention comprising two support elements which are a distance of 5 cm apart. This figure very clearly shows that the tilted position has virtually disappeared after the groups have stood on the device according to the invention. This means that the pelvises of the men and women were initially imbalanced and have been balanced by means of the device according to the invention.

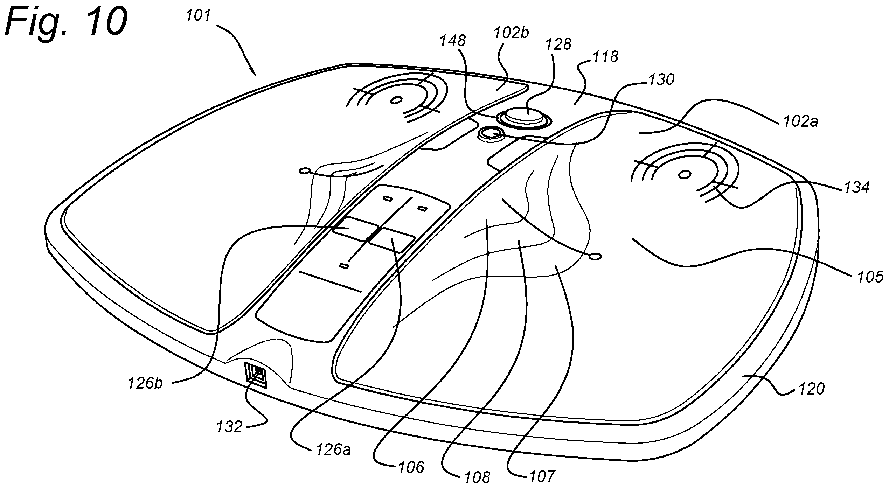

Fig. 10 shows a second embodiment of the invention in perspective view. Like elements to those of the first embodiment are denoted by similar reference numerals preceded by 100.

The device 101 comprises a treatment plate 120 on which are located first and second support elements 102a, 102b, each having a contoured surface 105. The contoured surfaces include a first elevation 106, a second elevation 107 and an intermediate part 108. Heel indicators 134 are also provided. The support elements 102a, b are separated from each other by a spacer 118. The spacer 118 is provided with left and right displays 126a, 126b, a power button 128 and an audio signal 130. On a front side of the device 101 is provided a communications port 132.

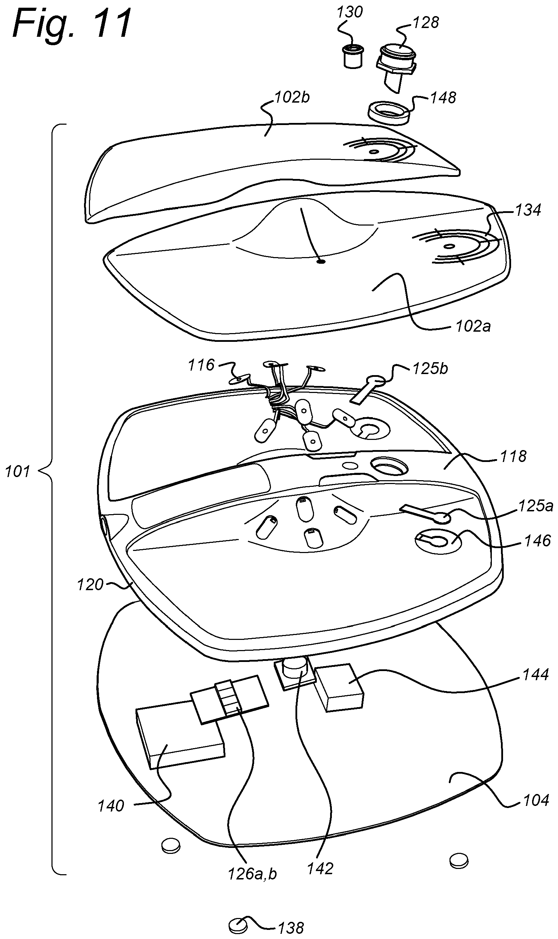

Fig. 11 shows the device 101 of Fig. 10 in exploded view showing the treatment plate 120 with the support elements 102a, b removed. Beneath the treatment plate 120 is provided a base plate 104 having rubber feet 138. The base plate 104 is of metal and sufficiently rigid to support a large person without deformation. Between the base plate 104 and the treatment plate 120 are provided the electronic components required for operation of the device 101. In assembled position, these components are retained within the spacer 118 and include an electronic control unit 140, displays 126a, b, vibration motor 142 and rechargeable power supply 144. Other components and connections may be present as required but are not otherwise relevant to the present description.

The treatment plate 120 itself is of moulded plastic material. Above the treatment plate 120 are located a plurality of sensors. These include pressure sensors 116 and left and right weight sensors 125a, b. The sensors 116, 125a, b are provided in recesses 146 formed in the treatment plate 120.

The left and right weight sensors 125 a, b are located at a position directly below the heel indicators 134. These sensors are covered over and held in place by the first and second support elements 102a, b respectively. These are formed of urethane rubber although other similar materials are also suitable. Also shown are the power button 128 and audio signal 130, including an LED ring 148 surrounding the power button 128, lighting in response to activation of the power button.

Operation of the device 101 according to Fig. 10 takes place as follows. A user or professional actuates the power button 128, causing initiation of the electronic control unit 140 and lighting of the LED ring 148. It will be understood that the power button 128 may also be omitted and the device may activate directly once a user steps onto the treatment plate 120. A user then stands onto the treatment plate 120, placing the feet apart with the heels centered on the heel indicators 134 and the feet pointing directly forwards. The left and right weight sensors 125a, b measure the weight of the user as exerted by the respective heel and output a value to the electronic control unit 140. This displays values on the left and right displays 126a, 126b representative of the respective weights measured, whereby a user can see the difference between the readings. The user then moves to the treatment position by placing the feet together with their medial edges against the spacer 118 and the arches on the first elevation 106, second elevation 107 and intermediate part 108. The contoured surface 105 lifts the arches but has a tendency to push the feet outwards, The rubber material of the contoured surface 105 assists in preventing the feet from sliding. Once the feet are correctly positioned above the pressure sensors 116 and each pressure sensor encounters a load of at least 100 grams, the electronic control unit 140 provides a signal to the audio signal 130 which emits a sound. The sound has been chosen to have a frequency of around 3000 Hz, which has been found conducive to good feedback and a relaxation of the muscles surrounding the spine and pelvis. Although not shown, the sensitivity of the pressure sensors may be adjusted to values above or below 100 grams depending on whether a user has high or low arches.

In order to perform the rebalancing exercise, the user must then bend the knees alternately while keeping the arches in contact with the contoured surface 105 and adequate pressure on the pressure sensors 116. This should be performed twice for each knee. During this procedure, the electronic control unit 140 controls the vibration motor 142 to oscillate, causing a vibratory signal to be emitted and experienced by the user through his feet. This signal is continued for approximately 10 seconds, which is normally adequate for performing the knee flexure. If the feet lose contact with the pressure sensors 116, the audio signal 130 stops, as does the vibration and the user must restart the procedure from the beginning.

After completion of the treatment, the user steps back onto the heel indicators 134 and is measured again. The respective values of the left and right weight sensors 125a, b are displayed on the left and right displays 126a, 126b and are also stored in a memory of the electronic control unit 140. If desired, the electronic control unit 140 can output the results to an external computer via the communications port 132.

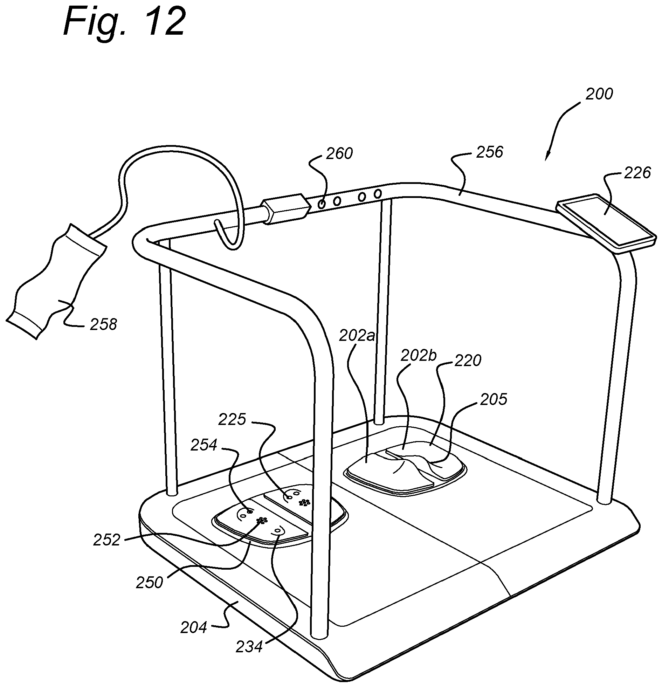

Fig. 12 shows a treatment installation according to a further embodiment of the invention. According to Fig. 12, the treatment installation 200 comprises a base plate 204 on which is provided a treatment plate 220 similar to that of Fig. 9. The treatment plate 220 includes first and second support elements 202a, 202b having contoured surfaces 205 and contact sensors. The treatment plate 220 differs from the device of Fig. 9 by the absence of the heel indicators and weight sensors (although these could also be provided). Also provided on the base plate 204 is a measurement plate 250. Measurement plate 250 is of a similar dimension to the treatment plate 220 but is generally flat. It includes heel indicators 234 identifying the locations of weight sensors 225. Additional weight sensors 225 are also provided at the front of the measurement plate at a position corresponding to the ball of a user's foot. Beneath the arch region, the measurement plate 250 is provided with a number of laser height sensors 252, able to measure the height of the arch above the measurement plate 250. These laser sensors 252 are otherwise conventional and are not further described in detail. In the forefoot region there is provided an infrared temperature sensor 254, capable of determining a reading indicative of blood flow rate. Other sensors may be provided as required.

The installation 200 is also provided with a hand rail 256 surrounding the base 204 at a height suitable to be grasped by a user. Display 226 in the form of a tablet computer is mounted on the hand rail 256 where it can be operated by a user or operator. The display 226 is connected to the communications port on the treatment plate 220 and to a similar communications port on the measurement plate 250. Also mounted on the hand rail 256 are a number of other sensors including blood pressure meter 258 and pulse-rate sensors 260. The later are mounted ahead of the treatment plate 220 at a position where they can be grasped by a user during treatment. It will be understood that the display 226 may provide all of the electronic control unit and that this need no longer be located in the treatment plate itself.

Operation of the installation 200 is similar to that described above, except that a user initially stands on the measurement plate 250 where all available measurements are made, in particular, the weight distribution between front and rear, left and right feet using pressure sensors 225. Additionally the height of the arches is measured using the height sensors 252. On the basis of these measurements, the sensitivity of the contact sensors monitoring the position of the user's arches on the treatment plate 220 may be adjusted to allow for the height of the arch. The user then stands onto the treatment plate 220 with the arches in contact with the contoured surfaces 205 and performs the knee flexures as described above. Correct performance of the treatment is accompanied by audible and vibratory feedback as described above. Changes in the user's heart rate during and after the treatment can be monitored using the pulse-rate sensors 260. After completion of the treatment, the user stands again onto the measurement plate 250 and the measurements are repeated. The display 226 provides a before and after analysis of the results.

Fig. 13 shows a bar graph which plots the average blood flow rate in the posterior tibial artery, using a HiDop® 300 Dopler probe available from Medizintechnik Basler AG for a test group of individual users of the device of Fig. 10. The test group comprised 80 persons, composed of 23 women and 57 men with an average age of 42.6. Measurements were initially performed on 76 individuals in a rest condition. The average values for the group for the right (light) and left (dark) legs are given by the bars marked A, with the values being given in KHz. The bars marked B give the values measured for the same individuals after performing 4 knee flexions according to the treatment protocol on the floor without use of the present invention. The bars marked C give the values measured for the same individuals after performing 4 knee flexions according to the treatment protocol on device according to Fig. 10. The values are considerably higher than those of bars A and B, indicating an increase in blood circulation. A total of 52 individuals were re-measured between 2 and 4 weeks after the first treatment. The bars marked D give the average values measured for these 52 individuals at rest.

Thus, the invention has been described by reference to certain embodiments discussed above. It will be recognized that these embodiments are susceptible to various modifications and alternative forms well known to those of skill in the art, without departing from the scope of the invention. Accordingly, although specific embodiments have been described, these are examples only and are not limiting upon the scope of the invention.

## Claims

1. Device (1) for rebalancing the pelvis, comprising first and second support elements (2), and a base (4), wherein the support elements (2), comprise:
a first side (3), the shape of which is adapted to rest on the base (4), and
a second side (5), the shape of which is such that when the first side (3) rests on the base (4), a first elevation (6), a second elevation (7) and an intermediate part (8) are formed, wherein the first elevation (6) comprises an upper surface with a surface area of 0.5 - 5 cm² and is higher than the second elevation (7), which comprises an upper surface with a surface area of 5 - 16 cm², the intermediate part (8) provides a gradual transition from the first elevation (6) to the second elevation (7), and the intermediate part (8) comprises an upper surface (9) which is inclined with respect to the base (4) with an angle of inclination (α) of 30 - 65 degrees, such that in a position of use for a user standing on the base (4) the support elements (2) are each situated underneath a large part of the arch (12) of a user's foot (10) in such a way that the first elevation (6) is situated underneath a first foot arch part (13), which first foot arch part (13) is situated in the centre on the medial side of the foot arch, and the second elevation (7) is situated underneath a second foot arch part (14),
**characterized in that**
the base comprises a first plate part (20a) for forming the base for the first support element (2a) and a first foot, and a second plate part (20a) for forming the base for the second support element (2b) and a second foot, and a first weighing unit (25a) and a second weighing unit (25b) are operatively connected to the first plate part (20a) and the second plate part (20b), respectively; and
each support element comprises a plurality of pressure sensors for determining a contact between the arch and the support element and an indicator operatively connected to the sensors for providing feedback of the contact between the arch and the support element, indicative of a correct position of the feet.

2. Device (1) according to Claim 1, **characterized in that** it comprises deforming means for moulding the support elements to a desired shape by deforming a deformable part, such as by pneumatic or hydraulic means.

3. Device (1) according to Claim 1, **characterized in that** it comprises a spacer (18) between the two support elements (2) of 0.5-15 cm.

4. Device (1) according to one of the preceding claims, **characterized in that** a pressure sensor (16) is incorporated at least in the first elevation (6), preferably each support element comprises at least three pressure sensors, activated by low pressure contact with the arch of a user's foot.

5. Device (1) according any preceding claim, **characterized in that** the first and second weighing units comprise first and second heel pressure sensors located on the first and second plate parts at a position that is spaced outwardly from the support elements.

6. Device (1) according to any preceding claim, **characterized in that** it further comprises a display for providing a readout from the first and second weighing units providing an indication of the difference therebetween.

7. Device (1) according to one of the preceding claims, **characterized in that** it also comprises a height sensor for determining the position of the first foot arch part (13) if the individual were to stand on the base (4) without being in contact with the support elements (2).

8. Device (1) according to one of the preceding claims, **characterized in that** it also comprises vibrating means for causing the support elements (2) to vibrate, the sensory feedback signal is a vibration provided in response to completion of a required action.

9. Device (1) according to one of the preceding claims, **characterized in that** it also comprises means for determining the body temperature.

10. Device (1) according to one of the preceding claims, **characterized in that** the sensory feedback signal is an audible or vibratory tactile signal for indicating the correct placement of a user's feet on the support elements (2), wherein the signal is preferably audible with a frequency between 50 Hz and 5000 Hz, more preferably between 2800 Hz and 2900 Hz.

11. Device (1) according to one of the preceding claims, **characterized in that** it further comprises an electronic control unit including a memory for controlling operation of the device and storing device parameters and treatment parameters and preferably a communication port for communicating with a remote computer.

12. Device according to claims 6, 8 10 and 11, **characterized in that** the electronic control unit is programmed to follow a treatment protocol comprising:
displaying respective heel weight readings when a user initially stands on the base with heels spaced apart and in contact with heel sensors;
emitting an audible signal when a user stands on the base with arches in contact with the support elements;
providing a tactile vibration during a period of at least 5 seconds while a user alternately bends a first and second knee four times and the arches remain in contact with the support elements.

13. Device (1) according to one of the preceding claims, **characterized in that** the base comprises a treatment plate on which the support elements are provided and a measurement plate provided with sensors for measuring anatomical parameters preferably selected from the group consisting of: blood flow rate, blood pressure, weight, weight distribution, arch height, temperature and pulse rate.

14. Device (1) according to one of the preceding claims, **characterized in that** it further comprises a hand rail at a height above the base for gripping by a user while standing on the base.

15. A device for rebalancing the pelvis according to claim 1, **characterized in that** the first and second support elements (2) are mounted on the base, and the support elements (2), which comprise contoured surfaces, preferably anti-slip surfaces, are arranged to support beneath the arches of a user's feet when standing on the support elements with the medial edges of the feet generally parallel and spaced by between 4 cm and 15 cm.

## Patentansprüche

1. Vorrichtung (1) zur Rekompensation des Beckens, mit ersten und zweiten Stützelementen (2) sowie einer Basis (4), wobei die Stützelemente (2) umfassen:
eine erste Seite (3), deren Form ausgestaltet ist, um auf der Basis (4) aufzuliegen, und
eine zweite Seite (5), deren Form so ist, dass dann, wenn die erste Seite (3) auf der Basis (4) aufliegt, eine erste Erhebung (6), eine zweite Erhebung (7) und ein zwischenliegender Abschnitt (8) gebildet werden, wobei die erste Erhebung (6) eine obere Fläche mit einem Flächengebiet von 0,5 - 5 cm² hat und höher ist als die zweite Erhebung (7), die eine obere Fläche mit einem Flächengebiet von 5 - 16 cm² hat, wobei der zwischenliegende Abschnitt (8) einen allmählichen Übergang von der ersten Erhebung (6) zur zweiten Erhebung (6) bildet, und wobei der zwischenliegende Abschnitt (8) eine obere Fläche (9) hat, die bezüglich der Basis (4) mit einem Neigungswinkel (α) von 30 - 65 Grad geneigt ist, so dass in einer Position zur Verwendung für einen Benutzer, der auf der Basis (4) steht, sich die Stützelemente (2) jeweils unter einem großen Teil des Gewölbes (12) des Fußes (10) eines Benutzers befinden, in einer solchen Weise, dass sich die erste Erhebung (6) unter einem ersten Fußgewölbeteil (13) befindet, wobei sich das erste Fußgewölbeteil (13) im Zentrum an der medialen Seite des Fußgewölbes (12) befindet, und sich die zweite Erhebung (7) unter einem zweiten Fußgewölbeteil (14) befindet,
**dadurch gekennzeichnet, dass**
die Basis ein erstes Plattenteil (20a) zum Bilden der Basis für das erste Stützelement (2a) und einen ersten Fuß sowie ein zweites Plattenteil (20a) zum Bilden der Basis für das zweite Stützelement (2b) und einen zweiten Fuß aufweist, und eine erste Wiegeeinheit (25a) und eine zweite Wiegeeinheit (25b) funktional mit dem ersten Plattenteil (20a) bzw. mit dem zweiten Plattenteil (20b) verbunden sind; und
jedes Stützelement eine Mehrzahl von Drucksensoren, um einen Kontakt zwischen dem Gewölbe und dem Stützelement zu bestimmen, und einen Indikator aufweist, der funktional mit den Sensoren verbunden ist, um ein Feedback des Kontakts zwischen dem Gewölbe und dem Stützelement zu bewirken, wodurch eine korrekte Position der Füße angegeben wird.

2. Vorrichtung (1) nach Anspruch 1, **dadurch gekennzeichnet, dass** diese Verformungsmittel zum Formen der Stützelemente in eine gewünschte Form durch Verformen eines verformbaren Teils, beispielsweise durch pneumatische oder hydraulische Mittel, aufweist.

3. Vorrichtung (1) nach Anspruch 1, **dadurch gekennzeichnet, dass** diese ein Abstandselement (18) zwischen den beiden Stützelementen (2) von 0,5 - 15 cm aufweist.

4. Vorrichtung (1) nach einem der vorhergehenden Ansprüche, **dadurch gekennzeichnet, dass** ein Drucksensor (16) zumindest in der ersten Erhebung (6) eingebaut ist, wobei jedes Stützelement vorzugsweise mindestens drei Drucksensoren aufweist, die durch einen geringen Druckkontakt mit dem Gewölbe eines Fußes des Benutzers aktiviert werden.

5. Vorrichtung (1) nach einem der vorhergehenden Ansprüche, **dadurch gekennzeichnet, dass** das erste und zweite Wiegemittel erste und zweite Fersendrucksensoren aufweisen, die an dem ersten und zweiten Plattenteil an einer Position angeordnet sind, die nach außen gerichtet von den Stützelementen beabstandet sind.

6. Vorrichtung (1) nach einem der vorhergehenden Ansprüche, **dadurch gekennzeichnet, dass** diese außerdem eine Anzeige zum Bereitstellen einer Auslesung von der ersten und zweiten Wiegeeinheit umfasst, um eine Angabe der Differenz zwischen diesen bereitzustellen.

7. Vorrichtung (1) nach einem der vorhergehenden Ansprüche, **dadurch gekennzeichnet, dass** diese außerdem einen Höhensensor zum Bestimmen der Position des ersten Fußgewölbeteils (13) umfasst, wenn die Person auf der Basis (4) steht, ohne mit den Stützelementen (2) Kontakt zu haben.

8. Vorrichtung (1) nach einem der vorhergehenden Ansprüche, **dadurch gekennzeichnet, dass** diese außerdem Vibrationsmittel aufweist, um zu bewirken, dass die Stützelemente (2) vibrieren, wobei das sensorische Feedback-Signal eine Vibration ist, die in Reaktion auf den Abschluss einer erforderlichen Aktion bereitgestellt wird.

9. Vorrichtung (1) nach einem der vorhergehenden Ansprüche, **dadurch gekennzeichnet, dass** diese außerdem Mittel zur Bestimmung der Körpertemperatur umfasst.

10. Vorrichtung (1) nach einem der vorhergehenden Ansprüche, **dadurch gekennzeichnet, dass** das sensorische Feedback-Signal ein hörbares oder vibratorisch taktiles Signal ist, um die korrekte Platzierung der Füße eines Benutzers auf den Stützelementen (2) anzugeben, wobei das Signal vorzugsweise hörbar ist, mit einer Frequenz zwischen 50 Hz und 5000 Hz, vorzugsweise zwischen 2800 Hz und 2900 Hz.

11. Vorrichtung (1) nach einem der vorhergehenden Ansprüche, **dadurch gekennzeichnet, dass** diese außerdem eine elektronische Steuereinheit mit einem Speicher, um den Betrieb der Vorrichtung zu steuern und um Vorrichtungsparameter und Behandlungsparameter zu speichern, und vorzugsweise einen Kommunikationsanschluss aufweist, um mit einem entfernten Computer zu kommunizieren.

12. Vorrichtung nach den Ansprüchen 6, 8, 10 und 11, **dadurch gekennzeichnet, dass** die elektronische Steuereinheit programmiert ist, um einem Behandlungsprotokoll zu folgen, das umfasst:
Anzeigen der jeweiligen Fersengewichtswerte, wenn ein Benutzer anfänglich auf der Basis steht, wobei die Fersen voneinander beabstandet sind und mit den Fersensensoren in Kontakt stehen;
Ausgeben eines hörbaren Signals, wenn ein Benutzer auf der Basis steht, wobei die Fußgewölbe mit den Stützelementen Kontakt haben;
Bereitstellen einer taktilen Vibration während eines Zeitraums von mindestens 5 Sekunden, während ein Benutzer abwechselnd ein erstes und ein zweites Knie viermal beugt und die Gewölbe mit den Stützelementen in Kontakt bleiben.

13. Vorrichtung (1) nach einem der vorhergehenden Ansprüche, **dadurch gekennzeichnet, dass** die Basis eine Behandlungsplatte, auf der die Stützelemente vorgesehen sind, und eine Messplatte aufweist, die mit Sensoren versehen ist, um anatomischer Parameter zu messen, die vorzugsweise ausgewählt sind aus der Gruppe, die umfasst: Blutflussrate, Blutdruck, Gewicht, Gewichtsverteilung, Gewölbehöhe, Temperatur und Pulsfrequenz.

14. Vorrichtung (1) nach einem der vorhergehenden Ansprüche, **dadurch gekennzeichnet, dass** diese außerdem einen Handlauf in einer Höhe oberhalb der Basis zum Greifen durch einen Benutzer umfasst, während dieser auf der Basis steht.

15. Vorrichtung zur Rekompensation des Beckens nach Anspruch 1, **dadurch gekennzeichnet, dass** die ersten und zweiten Stützelemente (2) an der Basis angebracht sind, und die Stützelemente (2), die konturierte Flächen haben, vorzugsweise rutschfeste Flächen, so angeordnet sind, um von unten die Gewölbe der Füße eines Benutzers abzustützen, wenn dieser auf den Stützelementen steht, wobei die medialen Kanten der Füße im Wesentlichen parallel verlaufen und mit zwischen 4 cm und 15 cm beabstandet sind.

## Revendications

1. Dispositif (1) de rééquilibrage du bassin, comprenant des premier et second éléments de support (2), et une base (4), dans lequel les éléments de support (2) comprennent :
un premier côté (3) dont la forme est adaptée pour reposer sur la base (4), et
un second côté (5) dont la forme est telle que lorsque le premier côté (3) repose sur la base (4), une première élévation (6), une seconde élévation (7) et une partie intermédiaire (8) sont formées, dans lequel la première élévation (6) comprend une surface supérieure avec une superficie de 0,5 à 5 cm² et est plus élevée que la seconde élévation (7), qui comprend une surface supérieure avec une superficie de 5 à 16 cm², la partie intermédiaire (8) fournit une transition progressive de la première élévation (6) à la seconde élévation (7), et la partie intermédiaire (8) comprend une surface supérieure (9) qui est inclinée par rapport à la base (4) avec un angle d'inclinaison (α) de 30 à 65 degrés, de sorte que dans une position d'utilisation pour un utilisateur debout sur la base (4), les éléments de support (2) sont chacun situés sous une grande partie de la voûte plantaire (12) d'un pied d'utilisateur (10) de telle sorte que la première élévation (6) est située sous une première partie de voûte plantaire (13), laquelle première partie de voûte plantaire (13) est située au centre sur le côté médian de la voûte plantaire, et la seconde élévation (7) est située sous une seconde partie de voûte plantaire (14),
**caractérisé en ce que**
la base comprend une première partie de plaque (20a) pour former la base du premier élément de support (2a) et un premier pied, et une seconde partie de plaque (20a) pour former la base du second élément de support (2b) et un second pied, et une première unité de pesage (25a) et une seconde unité de pesage (25b) sont connectées de manière opérationnelle à la première partie de plaque (20a) et à la seconde partie de plaque (20b), respectivement ; et
chaque élément de support comprend une pluralité de capteurs de pression pour déterminer un contact entre la voûte plantaire et l'élément de support et un indicateur connecté de manière opérationnelle aux capteurs pour fournir une rétroaction du contact entre la voûte plantaire et l'élément de support, indiquant une position correcte des pieds.

2. Dispositif (1) selon la revendication 1, **caractérisé en ce qu'**il comprend des moyens de déformation pour mouler les éléments de support à une forme souhaitée en déformant une pièce déformable, comme via des moyens pneumatiques ou hydrauliques.

3. Dispositif (1) selon la revendication 1, **caractérisé en ce qu'**il comprend une entretoise (18) entre les deux éléments de support (2) de 0,5 à 15 cm.

4. Dispositif (1) selon l'une des revendications précédentes, **caractérisé en ce qu'**un capteur de pression (16) est incorporé au moins dans la première élévation (6), de préférence chaque élément de support comprend au moins trois capteurs de pression, activés par un contact basse pression avec la voûte plantaire d'un pied d'utilisateur.

5. Dispositif (1) selon l'une quelconque des revendications précédentes, **caractérisé en ce que** les première et seconde unités de pesage comprennent des premier et second capteurs de pression de talon situés sur les première et seconde parties de plaque dans une position qui est espacée vers l'extérieur à partir des éléments de support.

6. Dispositif (1) selon l'une quelconque des revendications précédentes, **caractérisé en ce qu'**il comprend en outre un affichage pour fournir une lecture à partir des première et seconde unités de pesage fournissant une indication de la différence entre elles.

7. Dispositif (1) selon l'une des revendications précédentes, **caractérisé en ce qu'**il comprend également un capteur de hauteur pour déterminer la position de la première partie de voûte plantaire (13) si l'individu devait se tenir debout sur la base (4) sans être en contact avec les éléments de support (2).

8. Dispositif (1) selon l'une des revendications précédentes, **caractérisé en ce qu'**il comprend également des moyens vibrants pour amener les éléments de support (2) à vibrer, le signal de rétroaction sensorielle étant une vibration fournie en réponse à l'achèvement d'une action requise.

9. Dispositif (1) selon l'une des revendications précédentes, **caractérisé en ce qu'**il comprend également des moyens de détermination de la température corporelle.

10. Dispositif (1) selon l'une des revendications précédentes, **caractérisé en ce que** le signal de rétroaction sensorielle est un signal tactile audible ou vibratoire pour indiquer le placement correct des pieds d'un utilisateur sur les éléments de support (2), dans lequel le signal est de préférence audible avec une fréquence comprise entre 50 Hz et 5 000 Hz, de manière plus préférée entre 2 800 Hz et 2 900 Hz.

11. Dispositif (1) selon l'une des revendications précédentes, **caractérisé en ce qu'**il comprend en outre une unité de commande électronique comprenant une mémoire pour commander un fonctionnement du dispositif et stocker des paramètres de dispositif et de paramètres de traitement et de préférence un port de communication pour communiquer avec un ordinateur distant.

12. Dispositif selon les revendications 6, 8 10 et 11, **caractérisé en ce que** l'unité de commande électronique est programmée pour suivre un protocole de traitement comprenant les étapes consistant à :
afficher des lectures de poids de talon respectives lorsqu'un utilisateur se tient initialement debout sur la base avec des talons espacés et en contact avec des capteurs de talon ;
émettre un signal audible lorsqu'un utilisateur se tient sur la base avec des voûtes plantaires en contact avec les éléments de support ;
fournir une vibration tactile pendant une période d'au moins 5 secondes pendant qu'un utilisateur plie alternativement un premier et un second genou quatre fois et que les voûtes plantaires restent en contact avec les éléments de support.

13. Dispositif (1) selon l'une des revendications précédentes, **caractérisé en ce que** la base comprend une plaque de traitement sur laquelle sont prévus les éléments de support et une plaque de mesure munie de capteurs pour mesurer des paramètres anatomiques choisis de préférence dans le groupe comprenant : débit sanguin, pression artérielle, poids, répartition du poids, hauteur de la voûte plantaire, température et fréquence du pouls.

14. Dispositif (1) selon l'une des revendications précédentes, **caractérisé en ce qu'**il comprend en outre une main courante à une hauteur au-dessus du socle pour une préhension par un utilisateur debout sur la base.

15. Dispositif de rééquilibrage du bassin selon la revendication 1, **caractérisé en ce que** les premier et second éléments de support (2) sont montés sur la base, et les éléments de support (2), qui comprennent des surfaces profilées, de préférence des surfaces antidérapantes, sont agencés pour soutenir sous les voûtes plantaires des pieds d'un utilisateur lorsqu'il se tient debout sur les éléments de support avec les bords médians des pieds généralement parallèles et espacés de 4 cm à 15 cm.
